(19) Europäisches Patentamt
European Patent Office
Office européen des brevets

(11) **EP 1 600 144 A1**

(12) **EUROPEAN PATENT APPLICATION**

(43) Date of publication:
**30.11.2005 Bulletin 2005/48**

(51) Int Cl.[7]: **A61K 7/021**

(21) Application number: **04253033.7**

(22) Date of filing: **24.05.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **THE PROCTER & GAMBLE COMPANY**
**Cincinnati, Ohio 45202 (US)**

(72) Inventors:
- **Elliott, Russell Phillip**
**Egham Surrey, TW20 9RJ (GB)**
- **Cantor, Jennifer Clare**
**Sunbury, Middlesex, TW16 7NW (GB)**

(74) Representative: **Wilding, Richard Alan et al**
**Procter & Gamble Technical Centres Limited**
**Patent Department**
**Rusham Park**
**Whitehall Lane**
**Egham, Surrey TW20 9NW (GB)**

(54) **Translucent cosmetic foundation**

(57) A cosmetic foundation composition is provided having a $\Delta L_T$ value greater than or equal to 3.5, preferably greater than or equal to 4.5, more preferably greater than or equal to 5.0, where $\Delta L_T$ is measured according to the following equation:

$$\Delta L_T = \Delta L_2 - \Delta L_1$$

Where:

$$\Delta L_1 = L_1^{110} - L_1^{15};$$

$$\Delta L_2 = L_2^{110} - L_2^{15}$$

$L_1$ is a measurement of $L^y$ taken on bare skin, to which no product has been applied;
$L_2$ is a measurement of $L^y$ taken on skin, to which product has been applied; and
$L^y$ is the luminosity value at a specific angle, y, from specular.

Fig.2

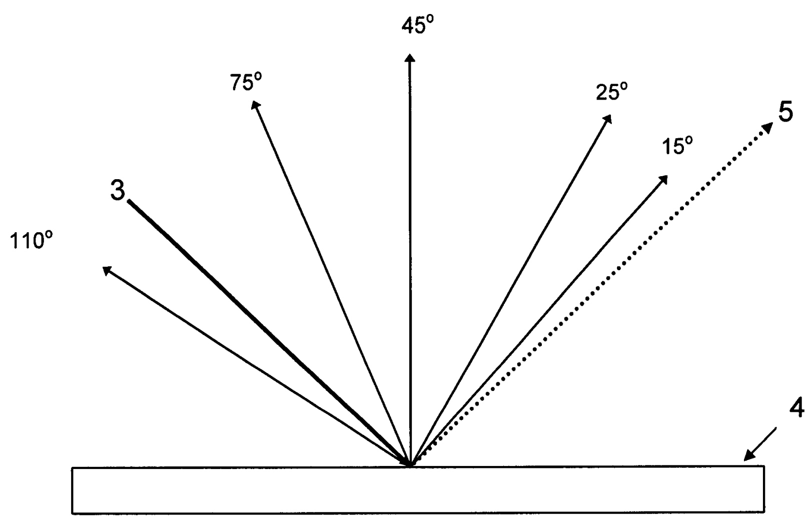


EP 1 600 144 A1

## Description

### FIELD OF THE INVENTION

The present application concerns translucent cosmetic foundation compositions.

### BACKGROUND OF THE INVENTION

Cosmetic skin foundations are known and have been for many years. Formulators of such foundations are faced with two apparently contradictory challenges - to increase the ability of the foundation to obscure the underlying skin while, at the same time, not bestowing an extremely artificial look on the skin. Preserving the natural appearance of the skin, however, is best achieved by employing a translucent product which allows the underlying skin to be seen - the apparent opposite of obscuring it. Not only that, but, with age, the translucency of skin diminishes, such that maturer users of cosmetic skin foundations generally express the desire for their foundations actually to increase apparent translucency. Currently, the only viable approach which addresses the issue of translucency of skin is to reduce the coverage, i.e. to reduce the concentration of metal oxide pigment in the composition, such that on application, less skin is actually obscured. At best this approach can approximately maintain the inherent translucency of the skin beneath, but such foundations are unable to provide sufficient coverage of the target surface. A small additional increase in apparent translucency may be achieved by adding a shiny emollient to the composition, but such increases are insufficient to meet the needs of the consumer.

The present invention addresses the above shortcomings in the prior art by providing a foundation that may provide high coverage of the underlying skin but retains and even enhances translucency to maintain a natural appearance.

### SUMMARY OF THE INVENTION

According to the invention a cosmetic foundation composition is provided having a $\Delta L_T$ value greater than or equal to 3.5, preferably greater than or equal to 4.5, more preferably greater than or equal to 5.0, where $\Delta L_T$ is measured according to the following equation:

$$\Delta L_T = \Delta L_2 - \Delta L_1$$

Where:
$\Delta L_1 = L_1^{110} - L_1^{15}$;
$\Delta L_2 = L_2^{110} - L_2^{15}$
$L_1$ is a measurement of $L^y$ taken on bare skin, to which no product has been applied;
$L_2$ is a measurement of $L^y$ taken on skin, to which product has been applied; and
$L^y$ is the luminosity value at a specific angle, y, from specular.

### BRIEF DESCRIPTION OF THE DRAWINGS

While the specification concludes with claims which particularly point out and distinctly claim the invention, it is believed the present invention will be better understood from the following description of preferred embodiments taken in conjunction with the accompanying drawings, in which like reference numerals identify identical elements and in which:

Fig. 1 is an illustration of a hiding power chart, discussed in greater detail below in relation to the method of calculating $\Delta L$.

Fig. 2 is a diagram illustrating how light incident at 45° to a skin surface reflects off that surface. Again, this diagram is discussed in greater detail below in relation to the method of calculating $\Delta L$.

### DETAILED DESCRIPTION OF THE INVENTION

All weights, measurements and concentrations herein are measured at 25°C on the composition in its entirety, unless otherwise specified.

Unless otherwise indicated, all percentages of compositions referred to herein are weight percentages of the total composition (i.e. the sum of all components present) and all ratios are weight ratios.

As used herein in relation to metal oxide particles, all weights of doping or coating materials are given as

percentages of the weight of the underlying metal oxide particle which is thus doped or coated. This definition applies even when the doping or coating material is, itself, a metal oxide. Thus, if the particles weigh x grammes and the coating or doping material weighs y grammes, the percentage weight of the coating or doping material is y/x * 100.

As used herein in relation to the cosmetic foundation composition, the percentage weight of the metal oxide sunscreen particles is the combined weight of the underlying metal oxide particle and any doping or coating divided by the weight of the entire cosmetic foundation composition. Thus, if the particles weigh x grammes, the coating or doping material weighs y grammes and the entire cosmetic foundation composition (including the coated or doped metal oxide particles) weighs z grammes, then the percentage weight of the metal oxide particle is (x + y)/z * 100.

Unless otherwise indicated, all polymer molecular weights are number average molecular weights.

Reference herein to the percentage weight of cross-linked organopolysiloxane elastomer in a composition is a reference to the percentage weight of solid organopolysiloxane elastomer in that composition.

Unless otherwise indicated, the content of all literature sources referred to within this text are incorporated herein in full by reference.

Except where specific examples of actual measured values are presented, numerical values referred to herein should be considered to be qualified by the word "about".

Translucency may be regarded as a measure of the way a surface upon which light is shone handles that light. To be more specific, it may be regarded as the difference in light intensity that an observer would see by observing the same part of a surface from different angles, when the surface is illuminated at a constant angle, such as 45° from the normal. Empirically, when under constant illumination at 45°, the most extreme differences are observed when the same part of the surface is observed from 15° (often called the "chroma band") and 110° (backscattered light) angles, measured from the specular reflectance band of the 45° incident light (not measured from the normal in this case). This is illustrated in Figure 2, discussed in greater detail hereinbelow. As a result, the difference in the light intensity measured at 15° and 110° may be regarded as a measure of translucence, with high positive values being indicative of high translucence and low positive or even negative values being indicative of low translucence surfaces.

Skin, especially young, healthy skin, has a relatively high translucence which means that there is a large difference between light intensity measured at 15° and 110° (more light is absorbed at 15° than 110°). This effect also may be explained by the composition of the skin, which allows light to enter and leave the skin resulting in different light path lengths according to the measurement angle - this follows from the Beer-Lambert Law.

Turning back to the issue in hand (the perception by consumers of skin coated with foundation), it is evident that covering skin with metal oxide particles has the effect of increasing light scattering in all directions, including at 15° and 110°, by preventing light from entering the skin. Since the path length of the light scattered at 15° is decreased more than that scattered at 110° (in fact, at 110°, there is little decrease in path length) the increase in light intensity at 15° is dramatically larger than at 110° such that the perceived translucence decreases with increasing coverage with metal oxide. A challenge faced by the present inventors was to retain or even increase the skin coverage by metal oxide particles while at the same time increasing perceived translucence.

The present inventors have unexpectedly established that the apparent or perceived luminosity achieved by cosmetic skin foundation may be increased by generating compositions having a reflective surface layer combined with islands of reduced opacity that allow a significant portion of incident light to reach, enter and then leave the skin to be received by an observer. Following luminosity measurements of currently marketed cosmetic foundation compositions, it has been established that none of those tested can achieve translucence, $\Delta L_T$, of 3.5 and above, preferably 4.5 and above, more preferably 5.0 and above, achieved by the present invention.

Advantageously, cosmetic foundation compositions according to the invention do not have a translucence, $\Delta L_T$, value greater than 30.

One way of increasing the translucence of skin covered with foundation is to include crosslinked organopolysiloxane elastomer in the composition. Without wishing to be bound by theory, such elastomers may create islands of reduced opacity in the dried-down film through which light may be transmitted to the skin. Although cross-linked organopolysiloxane elastomers are preferred for use herein, other materials which achieve the same effect may be employed in their stead.

Cross-linked organopolysiloxane elastomer may be present in cosmetic foundation compositions according to the invention in an amount from 0.01% to 15%, preferably from 1% to 10%, more preferably from 2 to 5% by weight of the cosmetic foundation composition.

The cosmetic foundation composition may comprise emulsifying cross-linked organopolysiloxane elastomer, non-emulsifying cross-linked organopolysiloxane elastomer or mixtures thereof. Preferably, the cross-linked organopolysiloxane elastomer comprises only non-emulsifying cross-linked organopolysiloxane elastomer. Non-emulsifying cross-linked organopolysiloxane elastomer may be present in an amount from 0.01 to 15%, preferably from 2 to 5% by weight of the cosmetic foundation composition.

As used herein, the term "non-emulsifying" when employed in relation to cross-linked organopolysiloxane elastomer includes cross-linked organopolysiloxane elastomer which comprise no polyoxyalkylene or polyglyceryl

units.

As used herein, the term "emulsifying" when employed in relation to cross-linked organopolysiloxane elastomer includes cross-linked organopolysiloxane elastomer which comprise at least one polyoxyalkylene (e.g., polyoxyethylene or polyoxypropylene) or polygyceryl unit.

No specific restriction exists as to the type of curable organopolysiloxane composition that can serve as starting material for the cross-linked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane; condensation-curing organopolysiloxane compositions which cure in the presence of an organotin compound or a titanate ester, by a condensation reaction between an hydroxyl-terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); peroxide-curing organopolysiloxane compositions which thermally cure in the presence of an organoperoxide catalyst; and organopolysiloxane compositions which are cured by high-energy radiation, such as by gamma-rays, ultraviolet radiation, or electron beams.

Preferred non-emulsifying organopolysiloxane elastomers are dimethicone/vinyl dimethicone crosspolymers. Such dimethicone/vinyl dimethicone crosspolymers are supplied by a variety of suppliers including Dow Coming (DC 9040, DC 9040 and DC 9045), General Electric (SFE 839 and the Velvasil range of products), Shin Etsu (KSG-15, 16, 18 [dimethicone/phenyl vinyl dimethicone crosspolymer]), and Grant Industries (Gransil(TM) line of materials), and lauryl dimethicone/vinyl dimethicone crosspolymers supplied by Shin Etsu (e.g., KSG-31, KSG-32, KSG-41, KSG-42, KSG-43, and KSG-44). Highly preferred non-emulsifying organopolysiloxane elastomers are DC9040 amd KSG15.

Cosmetic foundation compositions according to the invention may additionally comprise an dispersant, which has the function of wetting the metal oxide pigment particles to prevent agglomeration. With reference to the luminosity model discussed above, agglomeration can be considered to increase the luminosity at 15° (because there is an increase in random scattering of light from the surface), thereby decreasing the difference in light intensity between 15° and 110°, which in turn decreases the perceived translucence. Reducing or avoiding agglomeration counters this effect. Dispersants which may be employed according to the invention may be selected from the group consisting of classes A, B, and C, as defined below, or mixtures of these materials:

Class A materials are:

(a) are non-volatile; and

(b) have a viscosity less than or equal to 5cm$^2$/s (500 centistokes), preferably less than or equal to 1cm$^2$/s (100 centistokes), more preferably less than or equal to 0.5 cm$^2$/s (50 centistokes) at 25°C; and

(c) have a dielectric constant from 3.0 to 5.0, preferably from 3.5 to 5.0.

Examples of dispersants belonging to Class A include branched esters of diglycerin or triglycerin or the esters or 1,2,3,4 butane triol or erythritol, di erythritol or tri erthyritol. These esters must have at least one free hydroxyl group. Preferred dispersants in this class include erythrityl triethylhexanoate (available as Salacos E-38 from Nisshin Oilio) and Polyglyceryl-2 triisostearate (available as Cosmol 43V from Nisshin Oilio).

As used herein, the term "non-volatile" when employed in relation to dispersants includes materials that fulfil at least one of the following definitions: (a) the oil exhibits a vapour pressure of no more than about 0.2 mm Hg at 25°C and one atmosphere pressure; (b) the oil has a boiling point at one atmosphere of at least about 300°C.

Viscosity is measured on a Brookfield RV++ spindle at speed 100rpm and 25°C.

The dielectric constant of the dispersant was measured at 20°C using a Model 870 liquid dielectric constant meter manufactured by Scientifica in Princeton NJ. Readings were taken once equilibrium had been reached (as a rule, it took five minutes to achieve a constant value).

Class B materials adhere to the formula R - X - R', wherein R and R' are $C_6$-$C_{10}$ alkyl groups and X is an oxygen atom or a carbonate group, i.e. a group having the structure:

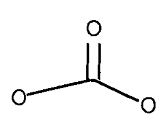

Preferably, X is a carbonate group.
R may be identical to or different from R'.

Preferably, R and R' are, independently, straight or branched chain $C_7$-$C_9$.
More preferably, both R and R' are $C_8$ alkyl groups.
More preferably still, both R and R' are 2-ethylhexyl groups and X is a carbonate group.

Commercially available dispersants falling within this class include TEGOSOFT DEC (Goldschmidt AG), CETIOL OE (Cognis AG) and CETIOL CC.

Class C comprises only isononyl isononanoate (available as Lanol 99 from Seppic).

In addition to wetting the metal oxide pigment particles to reduce or prevent agglomeration, dispersants may additionally have the effect of providing a reflective surface layer over the metal oxide particles. Without wishing to be bound by theory, it is believed that these materials may ensure that the applied cosmetic foundation composition has a microscopically smooth surface.

Cosmetic foundation compositions according to the invention may comprise from 0.1%wt to 20%wt, preferably from 0.5%wt to 10%wt, more preferably from 1%wt to 8%wt , more preferably still from 2%wt to 6%wt dispersant.

Cosmetic compositions according to the invention comprise metal oxide particles. Any suitable metal oxide that achieves the desired effect may be employed - for example, the metal oxide may provide a pigmentary effect, a sunscreening effect or a mixture of these effects. Preferably, the metal oxide particles are selected from the group consisting of titanium oxide, zinc oxide, zirconium oxide, yellow iron oxide, black iron oxide, red iron oxide, chromium oxide, chromium hydroxide, zirconium oxide and cerium oxide.

Advantageously, the metal oxide particles according to the invention have a number weighted average primary particle size from 10 to 500nm, preferably from 15 to 200nm, more preferably from 20-100nm.

As used herein, the term "primary particle size" means metal oxide crystal size, as determined by x-ray diffraction. It is based on measuring the broadening of the strongest rutile line.

Furthermore, the metal oxide particles may have a number weighted average secondary particle size from 0.005 to 100µm, preferably from 0.015 to 10µm, more preferably from 0.05 to 1µm. Sunscreening metal oxides may advantageously have a number weighted average secondary particle size from 100 to 250nm. Pigmentary metal oxides may advantageously have a number weighted average secondary particle size from above 250nm to 500nm.

The number weighted average secondary particle size is determined using a Nicomp 370 Sub Micron Particle Sizer.

Cosmetic foundation compositions according to the invention may comprise from 0.1 wt% to 45 wt% metal oxide particles, which may be selected from metal oxide pigments, metal oxide sunscreen particles and mixtures of these materials. Metal oxide pigments may be present from 0.05 wt% to 30 wt%, preferably from 1 wt% to 20 wt% of the cosmetic foundation composition. Metal oxide sunscreen particles may be present from 0.05 wt% to 15 wt%, preferably from 0.5 wt% to 10 wt%, more preferably from 1wt% to 5 wt% of the cosmetic foundation composition.

Minute metal oxide particles have a highly reactive surface that can cause unwarranted chemical or photochemical reactions. To counter this effect, it is known to dope these surfaces with one or more other materials such as silica, or metal oxides, such as alumina, to reduce the reactivity of the surface. This surface treatment may typically represent from 15 to 30% by weight of the metal oxide particle. Advantageously metal oxide particles comprised within cosmetic compositions according to the invention may be so-doped.

Advantageously, the metal oxide particles may be provided with a hydrophobic coating to improve the particles' dispersion in hydrophobic carrier medium. Advantageously, the metal oxide particles comprise from 2 to 25%, preferably from 5% to 15%, more preferably from 7% to 12% hydrophobic coating by weight of the metal oxide particles.

Advantageously, the hydrophobic coating may be made by applying a mixture of one or more of the following materials and isopropyl alcohol onto the metal oxide powder and drying at 150°C for 3 hours: reactive organo-polysiloxane, polyolefin (including polyethylene and polypropylene), hydrogenated lecithin and salts thereof, N-acylamino acid and salts thereof and dextrin fatty acid esters. Preferably, the reactive organo-polysiloxane comprises organo hydrogen polysiloxane, triorgano siloxy silicic acid and organopolysiloxane modified at both terminal ends with trialkoxy groups. Commercially available materials falling into the category of reactive organopolysiloxanes include KF-99, KF-9901, KF-7312F, KF-7312-J, KF-7312K, KF-9001, KF-9002, X-21-5249 and X-21-5250 manufactured by the Shin-Etsu Chemical Company Ltd; SH-1107, DC593, BY-11-015, BY-11-018 and BY-11-022 manufactured by Dow Coming Toray Silicone Co. Ltd.; TSF484, TSF483 and TSF4600 manufactured by Toshiba Silicone Co. Ltd.; FZ3704 and AZ6200 manufactured by Nippon Unicar Co. Ltd.

The hydrophobic coating is not limited to those described in the preceding paragraph and alternative hydrophobic coatings known to the skilled person may be employed instead. Such coatings may include trialkoyl isopropyl titanate, preferably triisostearoyl isopropyl titanate and perfluoro coatings, preferably polyperfluoroethoxymethoxy PEG-2 phosphate.

Cosmetic foundation compositions of the present invention may comprise an organic sunscreen. Suitable sunscreens may have UVA absorbing properties, UVB absorbing properties, or a mixture thereof.

As used herein, the term "UVA" refers to UV radiation having a wavelength of from about 320nm to about 400nm and the term "UVB" refers to UV radiation having a wavelength of from about 290nm to abut 320nm.

Advantageously, compositions of the present invention comprise from about 2% to about 20%, preferably from about 4% to about 14%, by weight, of UVA absorbing, organic sunscreen. Suitable sunscreens of this type may be found in the CTFA International Cosmetic Ingredient Dictionary and Handbook, $7^{th}$ edition, volume 2 pp. 1672, edited by Wenninger and McEwen (The Cosmetic, Toiletry, and Fragrance Association, Inc., Washington, D. C., 1997).

Suitable UVA absorbing, organic sunscreens may be selected from dibenzoylmethane derivatives, anthranilate derivatives such as methylanthranilate and homomethyl, 1-N-acetylanthranilate, and mixtures thereof.

Preferred UVA absorbing, organic sunscreens include those selected from 2-methyldibenzoylmethane, 4-methyldibenzoylmethane, 4-isopropyldibenzoylmethane, 4-tert-butyldibenzoylmethane, 2, 4-dimethyldibenzoylmethane, 2, 5-dimethyldibenzoylmethane, 4, 4'-diisopropylbenzoylmethane, 4-(1, 1-dimethylethyl)-4'-methoxydibenzoylmethane, 2-methyl-5-isopropyl-4'-methoxydibenzoylmethane, 2-methyl-5-tert-butyl-4'-methoxy-dibenzoylmethane, 2, 4-dimethyl-4'-methoxydibenzoylmethane, 2, 6-dimethyl-4'-tert-butyl-4'methoxydibenzoylmethane, and mixtures thereof. Preferred dibenzoyl sunscreen actives include those selected from 4-(1, 1-dimethylethyl)-4'-methoxydibenzoylmethane, 4-isopropyldibenzoylmethane, and mixtures thereof. A more preferred sunscreen active is 4-(1, 1-dimethylethyl)-4'-methoxydibenzoylmethane.

The sunscreen active 4-(1, 1-dimethylethyl)-4'-methoxydibenzoylmethane, which is also known as butyl methoxydibenzoylmethane or Avobenzone, is commercially available under the names of Parsol® 1789 from Givaudan Roure (International) S. A. (Basel, Switzerland) and Eusolex® 9020 from Merck & Co., Inc (Whitehouse Station, NJ). The sunscreen 4-isoproplydibenzoylmethane, which is also known as isopropyldibenzoylmethane, is commercially available from Merck under the name of Eusolex® 8020.

The compositions of the present invention may additionally comprise a UVB absorbing, organic sunscreen. Advantageously, compositions according to the invention may comprise from about 0.1% to abut 16%, preferably from about 0.1% to about 12%, and more preferably from about 0.5% to about 8% by weight, of UVB absorbing, organic sunscreen.

Suitable UVB absorbing, organic sunscreens are selected from 2-ethylhexyl-2-cyano-3, 3-diphenylacrylate (referred to as octocrylene), cinnamates and their derivatives such as 2-ethylhexyl-p-methoxycinnamate and octyl-p-methoxycinnamate, TEA salicylate, octyldimethyl PABA, camphor derivatives and their derivatives, and mixtures thereof. Preferred organic UVB absorbing, organic sunscreens are 2-ethylhexyl-2-cyano-3, 3-diphenylacrylate (referred to as octocrylene), 2-ethylhexyl-p-methoxycinnamate, and mixtures thereof.

Highly advantageously, cosmetic foundation compositions according to the invention comprise from 0.1%wt to 6%wt 2-ethylhexyl-p-methoxycinnamate.

Advantageously, cosmetic foundation compositions according to the invention comprise an oil. Oil may be present in an amount from 1% to 80% by weight of the cosmetic foundation composition.

The oil may be selected from the group consisting of silicones, which may be cyclic or linear, functionalised or non-functionalised; organic oils including $C_{10}$ to $C_{30}$ branched, linear or cyclic alkanes or esters and mixtures thereof, but excluding isononyl isononanoate.

Preferred cyclic silicones which may be employed in cosmetic foundation compositions according to the invention correspond to the formula:

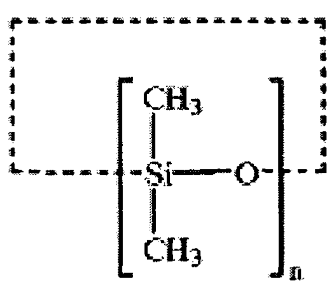

wherein n is from about 3 to about 8. Highly preferably, the cyclic volatile silicones are selected from cyclopentasiloxane, cyclohexasiloxane and mixtures thereof.

Preferred linear silicones which may be employed in cosmetic foundation compositions according to the invention correspond to the formula:

$$(CH_3)_3Si\text{-}O\text{-}[Si(CH_3)_2\text{-}O]_m\text{-}Si(CH_3)_3$$

wherein m is from about 1 to about 20 preferably from 3 to 12.

Linear silicones generally have a viscosity of less than about 50 centistokes, preferably less than 5 centistokes at 25°C; cyclic silicones generally have viscosities of less than about 10 centistokes at 25°C.

Examples of commercially available cyclic silicones include the following: Dow Coming 200, Dow Coming 244, Dow Coming 245, Dow Coming 344, and Dow Coming 345 (commercially available from Dow Coming Corp.); SF-1204 and SF-1202 Silicone Fluids (commercially available from G. E. Silicones), GE 7207 and 7158 (commercially available from General Electric Co.); and SWS-03314 (commercially available from SWS Silicones Corp.).

Preferred examples of linear dimethicones useful include DC200 5cst, DC1630 and DC 5-2117, More preferably, the linear dimethicone comprises DC 5-2117.

Preferred organic oils which may be included in cosmetic foundation compositions according to the invention are isohexadecane, isododecane and mixtures thereof.

Cosmetic foundation compositions according to the invention may be formulated as anhydrous products or as emulsions. If the cosmetic foundation compositions are formulated as emulsions, those emulsions may be water-in-oil (water-in-silicone) emulsions or oil-in-water (silicone-in-water) emulsions, but are preferably water-in silicone emulsions.

Advantageously, the cosmetic foundation compositions according to the invention are formulated as water-in-silicone emulsions that contain from 0.1 to 70%, preferably from 1 to 50%, more preferably from 5 to 40% water.

Cosmetic foundation compositions according to the invention, whether or not they are in the form of an emulsion, may comprise emulsifier. The emulsifier may be selected from the group consisting of nonionic, anionic, cationic, zwitterionic and amphoteric emulsifiers and mixtures thereof. Suitable emulsifiers are disclosed in McCutcheon's Detergents and Emulsifiers, North American Edition, pages 317-324.

In the event that the cosmetic foundation composition according to the invention is a water-in-silicone emulsion, then preferred emulsifiers are selected from the group consisting of polyoxyalkylene copolymers (also known as silicone polyethers), polyglyceryl copolymers and mixtures thereof. Polyoxyalkylene copolymers are described in detail in US 4,268,499. More preferred polyethers include PEG/PPG-18/18 Dimethicone available as blend with cyclopentasiloxane as DC5225C or DC5185; PEG 9 Dimethicone, available as KF6017 or KF6028 from Shin-Etsu. A preferred polyglyceryl emulsifier is available as KF6100 and KF6104 from Shin-Etsu Inc.

In one embodiment, it is preferred that cosmetic foundation compositions according to the invention comprise only polyglyceryl copolymer emulsifiers and no polyoxyalkylene emulsifiers. This is because polyoxyalkylene emulsifiers may break down to release ethylene glycol and aldehydes which may give rise to increased sensitivity on the skin of some consumers.

The total concentration of the emulsifier may be from 0.01 % to about 15%, more preferably from about 0.1% to about 10% of the formulation, even more preferably from 1.0% to about 5% and more preferably still from about 1.0% to about 3%, by weight of the composition.

Cosmetic foundation compositions according to the present invention may optionally contain spherical particles having an average particle diameter from 1 to 50 μm, preferably from 5 to 20 μm. As used herein in relation to the spherical particles, the particle diameter shall be understood to be that of primary particles.

Preferred spherical particles include, but are not limited, to polymeric particles chosen from the methylsilsesquioxane resin microspheres such as for example those sold by GE silicone under the name Tospearl 145A or Tospearl 2000; microspheres of polymethylmethacrylates such as those sold by Seppic under the name Micropearl M 100; the spherical particles of crosslinked polydimethylsiloxanes, especially such as those sold by Dow Coming Toray Silicone under the name Trefil E 506C or Trefil E 505C, sphericle particles of polyamide and more specifically Nylon 12, especially such as those sold by Atochem under the name Orgasol 2002D Nat C05, polystyerene microspheres such as for example those sold by Dyno Particles under the name Dynospheres, ethylene acrylate copolymer sold by Kobo under the name FloBead EA209 and mixtures thereof. Also found to be useful is Ronasphere LDP from Kobo Inc. Polyurethane particles BPD500 sold by Kobo Inc. may also be employed.

If present, the spherical particles may be included in the cosmetic foundation compositions according to the invention at a concentration of from about 0.01% to about 40%, more preferably from about 1% to about 10%, more preferably still from about 1%to about 5%.

Cosmetic foundation compositions according to the present invention may further comprise a skin-conditioning agent. These agents may be selected from humectants, exfoliants or emollients and may be present from about 0.01% to 30%, preferably from about 1% to about 20%, more preferably from about 1% to 10% by weight of the cosmetic foundation composition.

Humectants which may be included in cosmetic foundation compositions according to the invention include polyhydric alcohols such as glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerine and mixtures thereof. Most preferably the humectant comprises glycerine.

In addition, hydrophilic gelling agents such as those selected from the group consisting of the acrylic acid/ethyl acrylate copolymers, carboxyvinyl polymers (such as those sold by the B.F. Goodrich Company under the Carbopol trademark, polyacrylamides (such as those available from Seppic as Seppigel 305) and mixtures thereof may be included in the cosmetic foundation compositions according to the invention.

A variety of additional optional ingredients may be incorporated into the compositions of the present invention. Non-limiting examples of these additional ingredients include additional skin care actives such as peptides (e.g., Matrixyl [pentapetide derivative]), farnesol, bisabolol, phytantriol, urea, guanidine (e.g., amino guanidine); vitamins and derivatives thereof such ascorbic acid, vitamin A (e.g., retinoid derivatives such as retinyl palmitate or retinyl proprionate), vitamin E (e.g., tocopherol acetate), vitamin $B_3$ (e.g., niacinamide) and vitamin $B_5$ (e.g., panthenol) and the like and mixtures thereof; anti-acne medicaments (resorcinol, salicylic acid, and the like; antioxidants (e.g., phytosterols, lipoic acid); flavonoids (e.g., isoflavones, phytoestrogens); skin soothing and healing agents such as aloe vera extract, allantoin and the like; chelators and sequestrants; and agents suitable for aesthetic purposes such as essential oils, fragrances, skin sensates, opacifiers, aromatic compounds (e.g., clove oil, menthol, camphor, eucalyptus oil, and eugenol).

Method of calculating luminosity

All luminosity measurements made in the below-defined methods are carried out using an X-Rite™ MA68 II Spectrophotometer. For avoidance of doubt, however, any suitable multi-angle spectrometer could be used, provided it is properly calibrated with the same light source, the same incident light angle and the same reflectance angles (15° and 110° from specular, as defined in Figure 2) are used.

Foundation Standardisation

Colour at 45° incident light is measured on all products using the X-Rite™ MA68 II Spectrophotometer using a CMC (Colour Measurement Committee) tolerancing method which is considered most closely matched to the visual acceptability response of the human eye. Using the CMC equation (see British standard BS:6923) tolerance ellipsoids are calculated around the target in colour space allowing for the assessment of small colour differences between samples. The light source is a $D_{65}$ illuminant at a 10° standard observer. This light source is intended to represent average daylight and has a correlated colour temperature of approximately 6500 K (recognised as a standard by ISO 10526:1999).

With reference to Figure 1, which illustrates a hiding power chart (chart ref:301/2A supplied by Sheen Instruments Ltd.), product is applied to Region (1) at a thickness of 300µm and readings are taken over the middle of the hiding power chart in Region (2), where the black and white halves join, and are taken in the direction of the arrow shown on the right hand side of the figure.

All products to be tested must be comparable to shade 'Buff Beige' - shade #111 within the CoverGirl™ foundation palette - (L* = 68.72 a* = 11.47 b* = 19.49). L*, a* and b* refer to coordinates in 3D colour space, where L* indicates how light to dark the colour is, a* indicates the position on the red to green axis and b* indicates the position on the yellow to blue axis - these quantities are known to the skilled person in this area and need not be further defined.

The delta E values are calculated vs. this standard using the following equation:

$$\text{Delta E} = \sqrt{[(L - L^*)^2 + (a - a^*)^2 + (b - b^*)^2]}$$

For inclusion into testing, products must be no greater than a delta E of 0.5 vs. the standard. For avoidance of doubt, however, it is not necessary to use CoverGirl™ foundation in order to reproduce this method - any foundation achieving the Delta E value would be acceptable.

Translucency Measurement Method for a Given Product

Reflectance and Delta L are measured on the whole composition *in vivo* on human skin using the X-Rite™ MA68 II, 5-angle spectrophotometer, an industry standard device for analysing reflected light and colour. With reference to Figure 2, the spectrophotometer incident light (3) is at 45° to the skin surface (4), and it analyses reflected light at 15°, 25°, 45°, 75° and 110° away from the specular reflectance (5) (also at 45° to the surface and at 90° to the incident light).

As used herein, $L^y$ is the L-value measured at a specific angle, y, from specular.

As used herein, $L_1$, also referred to as the baseline measurement, is a measurement taken on bare skin, to which no product has been applied

As used herein, $L_2$, also referred to as post-application measurement, is a measurement taken on skin, to

which product has been applied.

Given the inherent variability of in vivo data, a base size of at least 15 test persons is required to provide statistically accurate data. Using multifactor analysis, it is possible to generate data showing highly significant differences between products using the present method. Prior to use, the instrument is calibrated using the white and black standards supplied with the machine. The test persons are Caucasian females aged 18-35 years.

The skin is illuminated with incident light at 45° from normal. The light source is a $D_{65}$ illuminant at a 10° standard observer (as described above). The skin is read three times without product to provide an arithmetical average base-line measurement ($L_1$). The base-line measurement of translucency ($\Delta L_1$) is defined as:

$$\Delta L_1 = L_1^{110} - L_1^{15}$$

The product is then applied to bare skin without markings (such as tattoos) on the forearm at a dosage of 15µl across a 3 cm x 3 cm square area (9 $cm^2$). The product is applied in an even film using a sweeping flat finger motion across a total of 15 swipes. After every 3 sweeps the direction of product wiping is rotated by 90°. The product is then allowed to dry on the skin for 5 minutes. An arithmetical average of three measurements ($L_2$) is then taken for each test person. The post-application measurement of translucency ($\Delta L_2$) is defined as:

$$\Delta L_2 = L_2^{110} - L_2^{15}$$

The base-line measurement, $\Delta L_1$, is then subtracted from the post-application measurement, $\Delta L_2$, to give the change in translucency ($\Delta L_T$)

$$\Delta L_T = \Delta L_2 - \Delta L_1$$

Translucent products have positive values of $\Delta L_T$. Highly translucent products according to the invention have $\Delta L_T$ values greater than or equal to 3.5, preferably greater than or equal to 4.5, more preferably greater than or equal to 5.0. As will be appreciated, for highly matte materials, this number may be negative, as these materials will actually reduce the amount of reflected light.

## Examples

The following examples further describe and demonstrate the preferred embodiments within the scope of the present invention. The examples are given solely for the purpose of illustration, and are not to be construed as limitations of the present invention since many variations thereof are possible without departing from its scope.

A liquid foundation of the present invention is prepared as follows: in a suitable vessel, water, glycerine, disodium EDTA and benzyl alcohol are added and mixed using conventional technology until a clear water phase is achieved. When the water phase is clear, the methylparabens are added and mixed again until clear. The resultant phase is mixed with a Silverson SL2T or similar equipment on high speed (8,000 rpm, standard head). In a separate vessel, the KSG21, DC245, Pigment dispersion, other oils, dispersant and the parabens are added and the mixture is milled using a Silverson SL2T on a high speed setting until a homogeneous mixture is created.

Following this step, the water phase and the silicone phase are combined and milled using the Silverson SL2T on a high speed setting until the water is fully incorporated and an emulsion is formed. The elastomer is then added and the mixture is mixed again using the Silverson on a high speed setting to generate the final product.

| Example # | 1 | 2 |
|---|---|---|
| Ingredient | | |
| DC9040 cross linked elastomer gel (1) | 25.0 | |
| KSG15 cross linked elastomer gel (2) | | 25.0 |
| Dimethicone copolyol cross-polymer (KSG21)(2) | 0.5 | 0.5 |

(1) Available from Dow Coming
(2) Available from Shin-Etsu

(continued)

| Example # | 1 | 2 |
|---|---|---|
| Ingredient | | |
| Decamethylcyclopentasiloxane (DC245)(1) | 11.0 | 9.0 |
| PEG/PPG18/18 Dimethicone & Cyclomethicone (DC5185) (1) | 2.0 | 2.0 |
| Octyl Methoxy cinnamate | 2.0 | 2.0 |
| Diethylhexyl carbonate (Tegosoft | 2.0 | 2.0 |
| DEC) (3) | | |
| Fibril coated sunscreen grade Titanium dioxide 50% dispersion in D5 SAS/TT0-S-3/D5 (4) | 5.5 | 5.5 |
| Pigmentary Titanium dioxide (9729) Coated with 2% methicone (5) | 9.00 | 9.00 |
| Black Iron Oxide coated with 2% methicone (5)* | 0.12 | 0.12 |
| Yellow Iron Oxide coated with 2% methicone (5)* | 1.2 | 1.2 |
| Red Iron Oxide coated with 2% methicone (5)* | 0.4 | 0.4 |
| Propylparabens | 0.1 | 0.1 |
| Ethylparabens | 0.1 | 0.1 |
| Methylparabens | 0.1 | 0.1 |
| Disodium EDTA | 0.1 | 0.1 |
| Benzyl alcohol | 0.25 | 0.25 |
| Sodium chloride | 2.00 | 2.00 |
| Glycerin | 10.00 | 10.00 |
| Water | qs | qs |
| | | |
| Luminosity, $\Delta L_T$ | 5.6 | 5.8 |

(1) Available from Dow Coming
(3) Available from Degussa
(4) Available from Miyoshi Kasei
(5) Available from Sensient
* these are added as slurries in cyclopentasiloxane (D5)

## Claims

1. A cosmetic foundation composition having a $\Delta L_T$ value greater than or equal to 3.5, preferably greater than or equal to 4.5, more preferably greater than or equal to 5.0, where $\Delta L_T$ is measured according to the following equation:

$$\Delta L_T = \Delta L_2 - \Delta L_1$$

Where:
$\Delta L_1 = L_1^{110} - L_1^{15}$;
$\Delta L_2 = L_2^{110} - L_2^{15}$
$L_1$ is a measurement of $L^y$ taken on bare skin, to which no product has been applied;
$L_2$ is a measurement of $L^y$ taken on skin, to which product has been applied; and
$L^y$ is the luminosity value at a specific angle, y, from specular.

2. The cosmetic foundation composition of claim 1, comprising from 0.01% to 15%, preferably from 1% to 10%, more

preferably from 2% to 5% by weight of the cosmetic foundation composition of cross-linked organopolysiloxane elastomer.

**3.** The cosmetic foundation composition of claim 2, wherein the cross-linked organopolysiloxane elastomer comprises non-emulsifying cross-linked organopolysiloxane elastomer.

**4.** The cosmetic foundation composition of claim 3, wherein the cross-linked organopolysiloxane elastomer consists only of non-emulsifying cross-linked organopolysiloxane elastomer.

**5.** The cosmetic foundation composition of any one of the preceding claims additionally comprising a dispersant.

**6.** The cosmetic foundation of claim 5, wherein the dispersant:

(a) is non-volatile; and
(b) has a viscosity less than or equal to 5cm$^2$/s (500 centistokes), preferably less than or equal to 1cm$^2$/s (100 centistokes), more preferably less than or equal to 0.5 cm$^2$/s (50 centistokes) at 25°C; and
(c) has a dielectric constant from 3.0 to 5.0, preferably from 3.5 to 5.0.

**7.** The cosmetic foundation of claim 5, wherein the dispersant adheres to the formula R - X - R', wherein R and R' are $C_6$-$C_{10}$ alkyl groups and X is an oxygen atom or a carbonate group.

**8.** The cosmetic foundation of claim 7, wherein X is a carbonate group.

**9.** The cosmetic foundation of claim 7 or 8, wherein R is different from R'.

**10.** The cosmetic foundation of claim 7 or 8, wherein R is identical to R'.

**11.** The cosmetic foundation of any one of claims 7 to 10, wherein R and R' are, independently, straight or branched chain $C_7$-$C_9$.

**12.** The cosmetic foundation of claim 10 or 11, wherein both R and R' are $C_8$ alkyl groups.

**13.** The cosmetic foundation of claim 12, wherein both R and R' are 2-ethylhexyl groups and X is a carbonate group.

**14.** The cosmetic foundation of claim 5, wherein the dispersant is isononyl isononanoate.

**15.** The cosmetic foundation of any one of claims 5 to 14, comprising from 0.1%wt to 20%wt, preferably from 0.5%wt to 10%wt, more preferably from 1%wt to 8%wt , more preferably still from 2%wt to 6%wt dispersant.

**16.** The cosmetic foundation composition of any one of the preceding claims additionally comprising metal oxide particles.

**17.** The cosmetic foundation composition of claim 16, wherein the metal oxide particles are selected from the group consisting of titanium oxide, zinc oxide, zirconium oxide, zirconium oxide, iron oxide and cerium oxide.

**18.** The cosmetic foundation composition of claim 16 or 17, comprising from 0.1 wt% to 45 wt% metal oxide particles

**19.** The cosmetic foundation composition of any one of claims 16 to 18, comprising from 0.05 wt% to 30 wt%, preferably from 1 wt% to 20 wt% of pigmentary metal oxide particles.

**20.** The cosmetic foundation composition of ant one of claims 16 to 19 comprising from 0.05 wt% to 15 wt%, preferably from 0.5 wt% to 10 wt%, more preferably from 1wt% to 5 wt% of metal oxide sunscreen particles.

**21.** The cosmetic foundation composition of any one of claims 16 to 20, wherein the metal oxide particles are provided with a hydrophobic coating.

**22.** The cosmetic foundation composition of any one of the preceding claims, additionally comprising an organic sunscreen.

**23.** The cosmetic foundation composition of claim 22, wherein the organic sunscreen is selected from the group consisting of UVA absorbing sunscreens, UVB absorbing sunscreens and mixtures thereof.

**24.** The cosmetic foundation composition of claim 23, comprising from 2 to 20%, preferably from 4 to 14% by weight of the cosmetic foundation composition of UVA absorbing sunscreen.

**25.** The cosmetic foundation composition of claim 23 or 24, wherein the UVA absorbing sunscreens are selected from the group consisting of 2-methyldibenzoylmethane, 4-methyldibenzoylmethane, 4-isopropyldibenzoylmethane, 4-tert-butyldibenzoylmethane, 2, 4-dimethyldibenzoylmethane, 2, 5-dimethyldibenzoylmethane, 4, 4'-diisopropylbenzoylmethane, 4-(1, 1-dimethylethyl)-4'-methoxydibenzoylmethane, 2-methyl-5-isopropyl-4'-methoxydibenzoylmethane, 2-methyl-5-tert-butyl-4'-methoxy-dibenzoylmethane, 2, 4-dimethyl-4'-methoxydibenzoylmethane, 2, 6-dimethyl-4'-tert-butyl-4'methoxydibenzoylmethane, and mixtures thereof.

**26.** The cosmetic foundation composition of any one of claims 23 to 25, wherein the UVA absorbing sunscreen comprises 4-(1, 1-dimethylethyl)-4'-methoxydibenzoylmethane.

**27.** The cosmetic foundation composition of claim 23, comprising from 0.1% to 16%, preferably from 0.1 to 12%, more preferably from 0.5% to 8% by weight of the cosmetic foundation composition of UVB absorbing sunscreen.

**28.** The cosmetic foundation composition of claim 27, wherein the UVB absorbing sunscreens are selected from the list consisting of 2-ethylhexyl-2-cyano-3, 3-diphenylacrylate, cinnamates and their derivatives, TEA salicylate, octyldimethyl PABA, camphor derivatives, and mixtures thereof.

**29.** The cosmetic foundation composition of claim 27 or 28, wherein the UVB absorbing sunscreen is 2-ethylhexyl-p-methoxycinnamate.

**30.** The cosmetic foundation composition of any one of the preceding claims, additionally comprising an emulsifier.

**31.** The cosmetic foundation composition of claim 30, wherein the emulsifier is selected from the group consisting of polyoxyalkylene copolymers, polyglyceryl copolymers and mixtures thereof.

**32.** The cosmetic foundation composition of any one of the preceding claims, additionally comprising spherical polymeric particles having an average particle diameter from 1 to 50 µm.

**33.** The cosmetic foundation composition of any one of the preceding claims, additionally comprising from 1% to 80% by weight of the cosmetic foundation composition of oil.

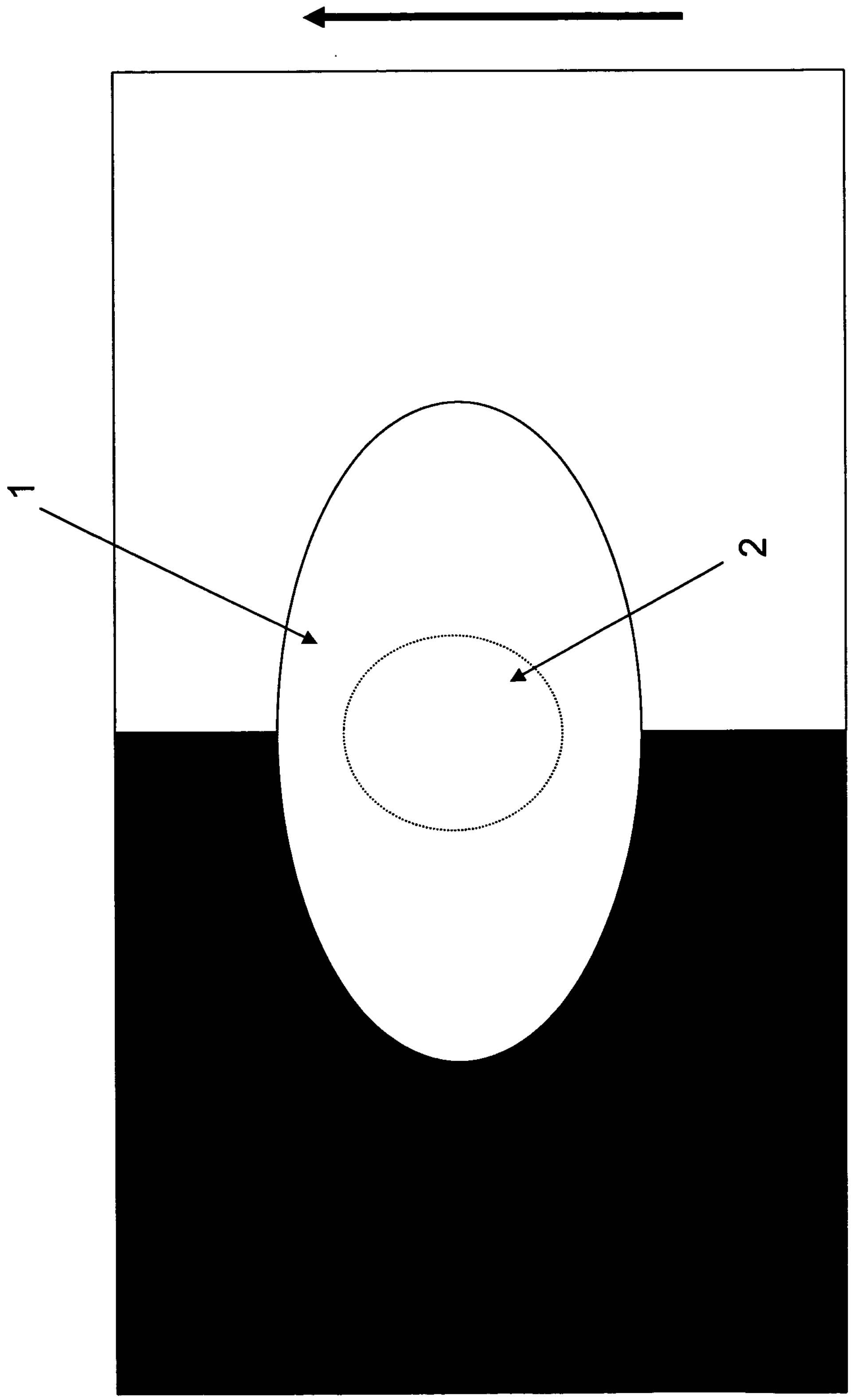


Fig.1

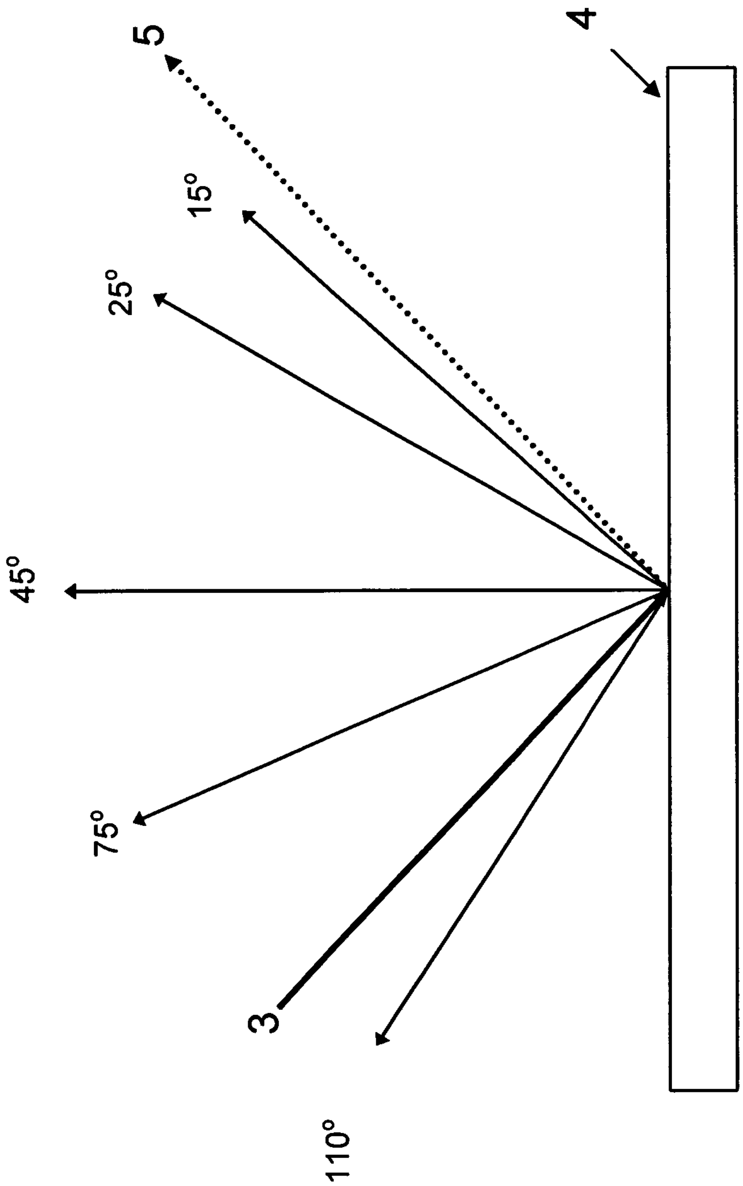


Fig.2

European Patent Office

# PARTIAL EUROPEAN SEARCH REPORT

which under Rule 45 of the European Patent Convention shall be considered, for the purposes of subsequent proceedings, as the European search report

Application Number
EP 04 25 3033

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 314 415 A (SHINETSU CHEMICAL CO) 28 May 2003 (2003-05-28) * sentence 23, paragraph 108 - paragraph 109; examples 4,23 * | 1-3, 16-33 | A61K7/021 |
| X | US 2002/028184 A1 (SUNKEL JORGE MAX ET AL) 7 March 2002 (2002-03-07) * claims; examples * | 1-3, 14-33 | |
| X<br>Y | US 6 258 345 B1 (CONTAMIN JEAN-CLAUDE ET AL) 10 July 2001 (2001-07-10) * examples * * column 6, line 4 * | 1-4, 15-33<br>1-33 | |
| Y | US 6 482 441 B1 (OHARA RYO ET AL) 19 November 2002 (2002-11-19) * column 37, paragraph 2 - paragraph 3; tables 8,13 * | 1-33 | |
| X | US 2002/028223 A1 (MOTLEY CURTIS BOBBY ET AL) 7 March 2002 (2002-03-07) * examples * | 1-4, 16-33 | |

-/--

TECHNICAL FIELDS SEARCHED (Int.Cl.7)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 5 November 2004 | Minas, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C07)

European Patent Office

# PARTIAL EUROPEAN SEARCH REPORT

Application Number
EP 04 25 3033

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 213 006 A (KANEBO LTD ; SHINETSU CHEMICAL CO (JP)) 12 June 2002 (2002-06-12) * paragraph [0057]; examples 1,6 * ----- | 1,2, 16-33 | |
| X | DATABASE CAPLUS [Online] CHEMICAL ABSTRACTS SERVICE, COLUMBUS, OHIO, US; ITANI, MAMORU ET AL VATTER, MICHAEL LEE ET AL BRUENING, STEFAN ET AL ELLIOTT, DAVID L. ET AL KAWASHIMA, FUMIKO ET AL AVENDANO, EST: "Manufacture of cosmetic compositions containing stably dispersed inorganic particles, and their use for sunscreens" XP002302812 retrieved from STN Database accession no. 2001:159411 * abstract * & JP 2001 058935 A2 (DAINIPPON KASEI CO., LTD., JAPAN; MIKUNI COLOR WORKS CO., LTD. THE PRO) 6 March 2001 (2001-03-06) ----- | 1-4, 16-21 | TECHNICAL FIELDS SEARCHED (Int.Cl.7) |
| X | DATABASE CAPLUS [Online] CHEMICAL ABSTRACTS SERVICE, COLUMBUS, OHIO, US; SATO, YUMIKO ET AL: "Transparent or translucent emulsified cosmetic stock" XP002302813 retrieved from STN Database accession no. 1998:293269 *IT* and * abstract * & JP 10 120524 A2 (KAO CORP., JAPAN) 12 May 1998 (1998-05-12) ----- -/-- | 1 | |

4

EPO FORM 1503 03.82 (P04C10)

European Patent Office

# PARTIAL EUROPEAN SEARCH REPORT

Application Number
EP 04 25 3033

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | WO 03/053396 A (COTY BV ; CERNASOV DOMINICA (US); MACCHIO RALPH (US); CHENG GUANG YU () 3 July 2003 (2003-07-03)<br>* page 5, paragraph 4 *<br>* page 9, line 15 - line 16; claims 1,2 *<br>----- | 1-4, 22-31,33 | |
| X | EP 0 197 870 A (MIFLEX SA)<br>15 October 1986 (1986-10-15)<br>* examples B,C; table II *<br>----- | 1-5,30, 31,33 | |
| A | ANONYMOUS: "Neue sonnenschutzformulierungen"<br>RESEARCH DISCLOSURE, KENNETH MASON PUBLICATIONS, HAMPSHIRE, GB,<br>vol. 466, no. 29, February 2003 (2003-02), XP007132193<br>ISSN: 0374-4353<br>----- | 7-13 | TECHNICAL FIELDS SEARCHED (Int.Cl.7) |

4

EPO FORM 1503 03.82 (P04C10)

European Patent Office

INCOMPLETE SEARCH
SHEET C

Application Number
EP 04 25 3033

Claim(s) searched completely:
2-4

Claim(s) searched incompletely:
5-33

Claim(s) not searched:
1

Reason for the limitation of the search:

Present claims 1 and 5-33 relate to cosmetic foundations defined by reference to a desirable characteristic or property, namely their "translucency" and defined by the use of parameters defining this "translucency". The claims cover all possible products having such a property, whereas the application provides support within the meaning of Article 84 EPC and disclosure within the meaning of Article 83 EPC for only a very limited number of such products. In the present case, the claims so lack support, and the application so lacks disclosure, that a meaningful search over the whole of the claimed scope is impossible. Independent of the above reasoning, the claims also lack clarity (Article 84 EPC). First, an attempt is made to define the product by reference to a result to be achieved, second, the use of these parameters in the present context is considered to lead to a lack of clarity within the meaning of Article 84 EPC. It is impossible to compare the parameter the applicant has chosen to employ with what is set out in the prior art. The lack of clarity is such as to render a meaningful complete search impossible. Consequently, the search has been restricted to compounds responsible for the translucency of the cosmetic foundation, namely to cross-linked organopolysiloxane elastomers [see first paragraph, p.5 and preferred examples (2nd paragraph, p.6)].

## ANNEX TO THE EUROPEAN SEARCH REPORT ON EUROPEAN PATENT APPLICATION NO. EP 04 25 3033

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2004

| Patent document cited in search report | | Publication date | Patent family member(s) | | | Publication date |
|---|---|---|---|---|---|---|
| EP 1314415 | A | 28-05-2003 | JP | 2002029918 | A | 29-01-2002 |
| | | | EP | 1314415 | A1 | 28-05-2003 |
| | | | WO | 0203928 | A1 | 17-01-2002 |
| | | | US | 2003082218 | A1 | 01-05-2003 |
| US 2002028184 | A1 | 07-03-2002 | AU | 7192801 | A | 21-01-2002 |
| | | | CA | 2412961 | A1 | 17-01-2002 |
| | | | CZ | 20030084 | A3 | 14-05-2003 |
| | | | EP | 1355624 | A2 | 29-10-2003 |
| | | | JP | 2004502715 | T | 29-01-2004 |
| | | | WO | 0203951 | A2 | 17-01-2002 |
| US 6258345 | B1 | 10-07-2001 | FR | 2768926 | A1 | 02-04-1999 |
| | | | BR | 9804138 | A | 14-12-1999 |
| | | | CA | 2245989 | A1 | 01-04-1999 |
| | | | DE | 69821180 | D1 | 26-02-2004 |
| | | | EP | 0908175 | A1 | 14-04-1999 |
| | | | ES | 2215282 | T3 | 01-10-2004 |
| | | | JP | 11158030 | A | 15-06-1999 |
| | | | PL | 328909 | A1 | 12-04-1999 |
| | | | US | 2002102284 | A1 | 01-08-2002 |
| | | | US | 2001026811 | A1 | 04-10-2001 |
| US 6482441 | B1 | 19-11-2002 | JP | 2001072527 | A | 21-03-2001 |
| | | | FR | 2795949 | A1 | 12-01-2001 |
| US 2002028223 | A1 | 07-03-2002 | AU | 7193101 | A | 21-01-2002 |
| | | | WO | 0203935 | A2 | 17-01-2002 |
| EP 1213006 | A | 12-06-2002 | AU | 6734700 | A | 26-03-2001 |
| | | | EP | 1213006 | A1 | 12-06-2002 |
| | | | CN | 1382033 | T | 27-11-2002 |
| | | | WO | 0115658 | A1 | 08-03-2001 |
| JP 2001058935 | A2 | 06-03-2001 | JP | 2001058935 | A | 06-03-2001 |
| JP 10120524 | A2 | 12-05-1998 | JP | 3484300 | B2 | 06-01-2004 |
| | | | JP | 10120524 | A | 12-05-1998 |
| WO 03053396 | A | 03-07-2003 | DE | 10164470 | A1 | 17-07-2003 |
| | | | WO | 03053396 | A1 | 03-07-2003 |
| EP 0197870 | A | 15-10-1986 | FR | 2579443 | A1 | 03-10-1986 |
| | | | EP | 0197870 | A1 | 15-10-1986 |

EPO FORM P0459

For more details about this annex : see Official Journal of the European Patent Office, No. 12/82